(19) Europäisches Patentamt
European Patent Office
Office européen des brevets

(11) Veröffentlichungsnummer: **0 289 881 A2**

(12) **EUROPÄISCHE PATENTANMELDUNG**

(21) Anmeldenummer: **88106460.4**

(51) Int. Cl.⁴: **C07D 237/32 , A61K 31/50**

(22) Anmeldetag: **22.04.88**

(30) Priorität: **02.05.87 DE 3714712**

(43) Veröffentlichungstag der Anmeldung:
**09.11.88 Patentblatt 88/45**

(84) Benannte Vertragsstaaten:
**AT BE CH DE ES FR GB GR IT LI LU NL SE**

(71) Anmelder: **ASTA Pharma AG**
**Weismüllerstrasse 45**
**D-6000 Frankfurt am Main 1(DE)**

(72) Erfinder: **Engel, Jürgen, Dr.**
**Erlenweg 3**
**D-8755 Alzenau(DE)**
Erfinder: **Scheffler, Gerhard, Dr.**
**Frankenwaldstrasse 19**
**D-6450 Hanau 6(DE)**

(54) **Neue 2-Aminoalkyl-4-bennzyl-1-(2H)-phthalazinon-Derivate.**

(57) Antiasthmatisch wirksame 2-Aminoalkyl-4-benzyl-1-(2H)-phthalazinon-Derivate der Formel

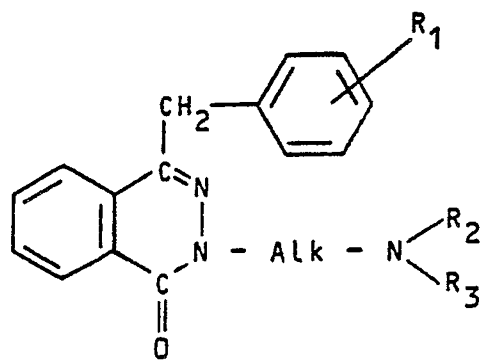

I

worin $R_1$ Wasserstoff, Fluor, Chlor, Brom, Trifluormethyl, $C_1$-$C_6$-Alkyl oder $C_1$-$C_6$-Alkoxy bedeuten, $R_2$ Wasserstoff, Phenyl, Benzyl, $C_1$-$C_6$-Alkyl oder $C_3$-$C_8$-Cycloalkyl bedeuten, $R_3$ Wasserstoff, einen $C_3$-$C_6$-Alkenylrest, einen $C_3$-$C_6$-Alkinylrest, einen Phenylrest oder einen Phenyl-$C_1$-$C_6$-alkylrest darstellt, der im Phenylteil gegebenenfalls durch Halogen, $NO_2$, $NH_2$, CN, OH, $C_1$-$C_6$-Alkyl, $C_1$-$C_6$-Alkoxy, $C_1$-$C_6$-Alkoxy-$C_1$-$C_6$-alkyl, $C_2$-$C_6$-Alkanoyloxy oder $C_2$-$C_6$-Alkanoylamino substituiert ist und Alk eine $C_2$-$C_6$-Alkylenbrücke darstellt, die auch eine Doppelbindung enthalten kann und Verfahren zu deren Herstellung.

EP 0 289 881 A2

Xerox Copy Centre

# Neue 2-Aminoalkyl-4-benzyl-1-(2H)-phthalazinon-Derivate

Die deutsche Auslegeschrift 1 046 625 betrifft ein Verfahren zur Herstellung basisch substituierter Phthalazone der allgemeinen Formel

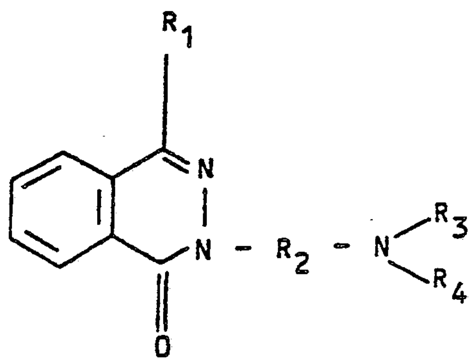

in der $R_1$ einen - gegebenenfalls im Kern substituierten - Aryl-oder Aralkylrest bedeuted, $R_2$ eine zweiwertige gerade oder verzweigte aliphatische Kette mit wenigstens 2 und höchstens 5 Kohlenstoffatomen darstellt und $R_3$ und $R_4$ niedermolekulare Alkylgruppen bedeuten, die gemeinsam mit dem Stickstoff Glieder eines heterocyclischen Ringes sein können, beziehungsweise deren Salze oder quaternärer Ammoniumverbindungen.

Für diese Verbindungen wird eine histaminolytische (Antihistamin-Wirkung), spasmolytische und lokalanästhetische Wirkung angegeben.

Demgegenüber zeichnen sich die erfindungsgemäßen Verbindungen der Formel I durch folgende überraschende Wirkung aus: Sie wirken antiasthmatisch, antiallergisch, Paf-antagonistisch (Paf = platelet activating factor, Mediator, der unter anderem Asthma auslöst) sowie Leukotrien-inhibierend.

Der Rest $R_1$ befindet sich vorzugsweise in 4-Stellung des Phenylringes; vorkommende $C_1$-$C_6$-Alkylgruppen, $C_1$-$C_6$-Alkoxygruppen, Alkenylgruppen oder Alkinylgruppen können gerade oder verzweigt sein, insbesondere bestehen diese Reste aus 1-4 beziehungsweise, falls sie ungesättigt sind, aus 3-4 C-Atomen. Falls $R_3$ eine Alkenyl-oder Alkinylgruppe ist, befindet sich zwischen der ungesättigten Bindung und dem Stickstoff mindestens ein gesättigtes C-Atom. Vorzugsweise befindet sich die ungesättigte Bindung in 2,3-Stellung oder 3,4-Stellung.

Bei dem $C_3$-$C_8$-Cycloalkylrest handelt es sich insbesondere um den Cyclopentylrest oder Cyclohexylrest.

Falls $R_3$ einen Phenyl-$C_1$-$C_6$-alkylrest darstellt, kann dieser einfach, zweifach oder dreifach durch die angegebenen Reste substituiert sein. Der Alkylteil dieses Phenylalkylrestes besteht vorzugsweise aus einem, zwei oder drei C-Atomen und kann gegebenenfalls auch verzweigt sein.

Die Alkylenbrücke Alk kann gerade oder verzweigt sein und besteht vorzugsweise aus zwei, drei oder vier C-Atomen. Falls diese Alkylenbrücke Alk eine Doppelbindung enthält, ist diese zur Gruppe $NR_2R_3$ isoliert, falls $R_2$ Wasserstoff bedeutet (das heißt nicht konjungiert zu dieser Gruppe). Vorzugsweise befindet sich zwischen einer solchen Doppelbindung und den beiden Stickstoffbindungen jeweils mindestens ein gesättigtes Kohlenstoffatom.

Besonders günstige Wirkungen besitzen zum Beispiel solche Verbindungen, wo die Reste $R_1$ bis $R_3$, Alk die folgenden Bedeutungen haben: $R_1$ = Fluor, Chlor oder Brom, insbesondere in 4-Stellung, vorzugsweise Fluor in 4-Stellung; $R_2$ = $C_1$-$C_6$-Alkyl, vorzugsweise Methyl; $R_3$ = Phenyl-$C_1$-$C_6$-alkyl, gegebenenfalls wie angegeben substituiert.

Bei den Substituenten des Phenyl-$C_1$-$C_6$-alkylrestes handelt es sich vorzugsweise um $C_1$-$C_4$-Alkylgruppen (insbesondere Methyl) oder um ein Halogen (zum Beispiel Cl, F) oder $C_1$-$C_4$-Alkoxygruppen (insbesondere Methoxygruppen). Die Substituenten im Phenylteil dieses Phenylalkylrestes befinden sich vorzugsweise in 2-Stellung, 3-Stellung, 4-Stellung oder 2,4-Stellung. Vorkommende Alkyl-, Alkoxy-, Alkanoyloxy-, Alkanoylamino-oder Alkoxy-alkylgruppen können gerade oder verzweigt sein. Alkyl-beziehungsweise Alkoxyreste bestehen vorzugsweise aus 1 bis 4 C-Atomen, die Alkanoylreste vorzugsweise aus 2 bis 4 C-Atomen.

Zu dem Verfahren a)

Das Verfahren kann ohne Lösungsmittel oder in einem geeigneten Lösungs-oder Dispergiermittel durchgeführt werden. Als Lösungs-oder Dispergiermittel kommen zum Beispiel in Betracht: Aromatische Kohlenwasserstoffe wie zum Beispiel Benzol, Mesitylen, Toluol, Xylol; Pyridin; niedere aliphatische Ketone wie zum Beispiel Aceton, Methyläthylketon; halogenierte Kohlenwasserstoffe wie zum Beispiel Chloroform, 1,2-Dichlorethan, Tetrachlorkohlenstoff, Chlorbenzol, Methylenchlorid; Ether wie zum Beispiel Tetrahydrofuran, Dioxan, Diisopropylether; Sulfoxyde wie zum Beispiel Dimethylsulfoxid; tertiäre Säureamide wie zum Beispiel Dimethylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol sowie Mischungen der genannten Mittel, gegebenenfalls auch mit Wasser. Falls in der Ausgangssubstanz II die Gruppe $-Alk-NHR_2$ beziehungsweise $-Alk-NHR_3$ ist, werden vorzugsweise dipolar aprotische Lösungsmittel wie Dimethylsulfoxid oder tertiäre Säureamide verwendet.

Die Reaktion wird beispielsweise bei Temperaturen zwischen 20 bis 200° C, vorzugsweise 40 bis 160° C oder auch 50 bis 120° C durchgeführt.
Wird ein Lösungs-beziehungsweise Dispersionsmittel ver wendet, arbeitet man häufig bei der Rückflußtemperatur dieses Mittels. Die Reaktion läuft häufig bereits auch schon bei Raumtemperatur ab, beziehungsweise bei einer Temperatur zwischen 40 bis 120° C.

Die Umsetzung wird vorteilhaft in Gegenwart von säurebindenden Mitteln wie Alkalicarbonaten, Pottasche, Soda, Alkaliacetaten, Alkalihydroxyden oder tertiären Basen (Triethylamin, Pyridin) durchgeführt.

Falls in der Ausgangssubstanz der Formel II der Rest X Wasserstoff ist, wird diese vorzugsweise in Form ihres Metallsalzes eingesetzt. Insbesondere kommen hier die Alkalisalze (Na, K, Li) in Frage.
Die Herstellung der Alkalisalze erfolgt beispielsweise mittels den entsprechenden Alkaliamiden, Alkalialkoholaten oder auch Alkalimetallen in einem Lösungsmittel (niederer Alkohol, aromatischer Kohlenwasserstoff) oder mit wäßrigem Alkali (zum Beispiel NaOH).

Falls Y eine veresterte Hydroxygruppe bedeutet, dann handelt es sich hierbei um reaktionsfähige Ester. Ein reaktionsfähiger Ester ist dabei zum Beispiel derjenige einer starken organischen oder anorganischen Säure, wie vor allem einer Halogenwasserstoffsäure, zum Beispiel der Chlor-, Brom-oder Jodwasserstoffsäure, oder einer Sulfonsäure, wie einer Aryl-oder $C_1$-$C_6$-Alkylsulfonsäure, zum Beispiel von niederen Alkylbenzolsulfonsäuren (p-Toluolsulfonsäure).

Nicht bekannte Ausgangsstoffe der Formel III können zum Beispiel analog Houben-Weyl, Methoden der Organischen Chemie, Band 5/3 (1962), Seite 503 ff., Band 6/2 (1963), Seite 475 ff. oder Band 9 (1955), Seite 426 erhalten werden. Ausgangsstoffe III der Formel $ZR_3$, worin Z zum Beispiel eine Hydroxygruppe ist, die durch einen Arylsulfonsäurerest verestert ist, können zum Beispiel aus den entsprechenden Alkoholen ($R_3OH$) durch Umsetzung mit den entsprechenden Arylsulfonsäurechloriden in bekannter Weise erhalten werden. In analoger Weise erhält man zum Beispiel aus den Alkoholen und Thionylchlorid oder Thionylbromid die entsprechenden Verbindungen mit Z = Cl oder Br. In analoger Weise können Ausgangsstoffe $Z-Alk-NR_2R_3$ aus den Alkoholen $HO-Alk-NR_2R_3$ durch Veresterung erhalten werden.

Herstellung von Ausgangsstoffen der Formel II: Nichtbekannte Ausgangsstoffe II, worin X die Gruppe $-Alk-NHR_2$ beziehungsweise $-Alk-NHR_3$ ist, können zum Beispiel analog dem in der Deutschen Auslegeschrift 1 046 625 beschriebenen Verfahren hergestellt werden, wobei in den hierzu verwendeten Halogeniden $Hal-Alk-NHR_2$ beziehungsweise $Hal-Alk-NHR_3$ das basische Stickstoffatom zweckmäßig durch eine Benzylgruppe geschützt ist. Diese Benzylgruppe wird dann in üblicher Weise abgespalten (zum Beispiel abhydriert). Ausgangsstoffe II, worin X die Gruppe Alk-Y ist, können zum Beispiel durch Umsetzung des entsprechenden Phthalazons mit einem Alkohol Hal-Alk-OH gemäß der deutschen Auslegeschrift 1 046 625 und anschließender Veresterung der Hydroxygruppe (zum Beispiel wie oben angegeben) erhalten werden.

Herstellung von Ausgangssubstanzen II, worin X Wasserstoff ist, sind beispielsweise herstellbar wie in der DAS 10 46 625 beschrieben wird, sowie analog wie bei den Beispielen angegeben wird; außerdem analog den Arbeitsweisen gemäß der DOS 36 34 942.9.

Zu dem Verfahren b)

Als reaktionsfähige Derivate der Carbonsäure der allgemeinen Formel IV kommen insbesondere die Säurehalogenide (-chloride, -bromide, -jodide), -ester (insbesondere mit $C_1$-$C_6$-Alkanolen; oder auch innere

Ester mit der enolisierten Ketogruppe (beispielsweise p-Chlor-benzyliden-phthalid) und -anhydride in Frage. Die Verbindungen der Formel IV sowie der entsprechenden Säurehalogenide und Ester mit $C_1$-$C_6$-Alkanolen können auch in der tautomeren cyclischen Form vorliegen. Diese cyclische Form wird durch folgende Formel ausgedrückt:

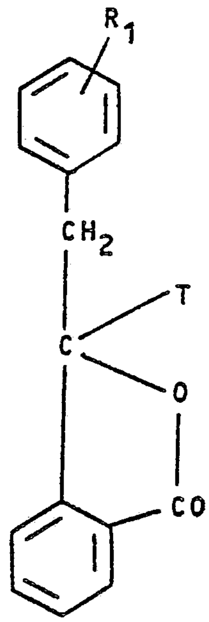

Hierbei bedeutet T die Hydroxygruppe, Halogen oder $C_1$-$C_6$-Alkoxy.

Die Umsetzung wird in An-oder Abwesenheit der üblichen Lösungs-und Hilfsmittel bei Temperaturen zwischen 40 und 200° C und in weitem pH-Bereich vom Sauren bis zum Alkalischen durchgeführt.

Als Lösungsmittel eignen sich zum Beispiel Wasser, aromatische Kohlenwasserstoffe wie zum Beispiel Benzol, Mesitylen, Toluol, Xylol; halogenierte Kohlenwasserstoffe wie zum Beispiel Chloroform, 1,2-Dichlorethan, Tetrachlorkohlenstoff, Chlorbenzol, Methylenchlorid; Ether wie zum Beispiel Tetrahydrofuran, Dioxan, Diisopropylether; Sulfoxyde wie zum Beispiel Dimethylsulfoxid; tertiäre Säureamide wie zum Beispiel Dimethylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol und Mischungen der genannten Mittel sowie auch tertiäre Amine, zum Beispiel Pyridin. Als Hilfsmittel können in Frage kommen Basen, Säuren und für diese Reaktionen übliche Kondensationsmittel.

Die Verbindung V kann auch in Form eines N-Acylderivats eingesetzt werden, das dann zuerst hydrolisiert wird und ohne weitere Reinigung oder Isolierung in demselben Reaktionsmedium sofort weiter mit der Verbindung IV umgesetzt wird.

Insbesondere kommen als Lösungsmittel tertiäre Säureamide (zum Beispiel Dimethylformamid), aromatische Kohlenwasserstoffe (zum Beispiel Toluol, niedere Alkohole) oder auch Wasser in Frage, wobei häufig in Gegenwart basischer Stoffe (zum Beispiel Alkalihydroxiden beziehungsweise Alkalialkoholaten) gearbeitet wird. Vorzugsweise wird bei Temperaturen zwischen 50 - 200° C, insbesondere 80 - 150° C gearbeitet.

Herstellung von Ausgangsverbindungen der Formel V: Solche Ausgangsstoffe können beispielsweise wie folgt erhalten werden:

Umsetzung von entsprechenden Oxoverbindungen der Formel $0 = Alk'$-$NR_2R_3$ mit Acetylhydrazin (zum Beispiel in einem aromatischen Kohlenwasserstoff (Toluol) zwischen 50 - 150° C) oder Benzoylhydrazin (zum Beispiel in einem aliphatischen Alkohol zwischen 20 - 100° C) zu den entsprechenden Hydrazonen, anschließende Reduktion derselben durch katalytische Hydrierung (zum Beispiel in Eisessig in Gegenwart von $PtO_2$ bei 5-6 bar) oder Reduktion mit einem komplexen Metallhydrid ($NaBH_4$) in einem inerten Mittel (niedere Alkohole wie Methanol oder Dioxan) und anschließende Abspaltung der Acylgruppe durch Hydrolyse mit verdünnter Salzsäure (20-37 %ig). Bei den Oxo-Verbindungen der Formel $0 = Alk'$-$NR_2R_3$ bedeutet $Alk'$ den Rest Alk, wobei das Kohlenstoffatom welches Alk mit dem Phthalazinon-Rest verbindet anstelle einer Wasserstoffbindung eine zweite Bindung mit dem Oxo-Sauerstoff hat.

Eine andere Möglichkeit zur Herstellung von Ausgangssubstanzen V ist beispielsweise die Umsetzung eines Amins der Formel $R_2R_3N$-Alk-$NH_2$ mit einem Keton (zum Beispiel Cyclohexanon) und Hydroxylamin-0-sulfonsäure analog Weygand-Hilgetag, Organisch-Chemische Experimentierkunst, 1970, Seite 602, Verlag Johann Ambrosius Barth, Leipzig.

Ausgangsstoffe der Formel IV können beispielsweise in bekannter Weise durch übliche Perkin-Synthese aus Phthalsäureanhydrid und der durch den Rest $R_1$ substituierten Phenylessigsäure erhalten werden.

Die Abspaltung einer Benzylgruppe (falls zum Beispiel $R_2$ und $R_3$ Benzyl ist, oder falls $R_2$ Benzyl ist und $R_3$ die angegebenen Bedeutungen hat) erfolgt in bekannter Weise in einem Lösungs-beziehungsweise Suspensionsmittel wie aromatischen Kohlenwasserstoffen (Benzol, Toluol, Xylol), aliphatischen Alkoholen (Äthanol, Propanol, Butanol), niederen aliphatischen Säureamiden (Dimethylformamid), Tetramethylharnstoff, Dimethylsulfoxyd , alicyclischen und cyclischen gesättigten Äthern (Diäthyläther, Dioxan) bei Temperaturen zwischen 20 bis 200° C, insbesondere 50 bis 140° C durchgeführt.
Die Abspaltung erfolgt zum Beispiel mittels Wasserstoff in Gegenwart üblicher Hydrierungskatalysatoren. Vorzugsweise werden metallische Hydrierungskatalysatoren wie Raney-Nickel-, Platin-oder Palladium-Katalysatoren, insbesondere Palladium-Katalysatoren verwendet.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern, wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen.

Die erfindungsgemäßen Verbindungen der Formel I können asymmetrische Kohlenstoffatome enthalten und werden dann in der Regel als Racemate erhalten. Solche Racemate können in an sich bekannter Weise beispielsweise durch fraktionierte Kristallisation der Salze von racemischen Verbindungen I mit optisch aktiven Säuren oder auch durch chromatographische Racemattrennung (siehe beispielsweise Angewandte Chemie 92/1 (1980) Seite 14) in die optisch aktiven Isomeren gespalten werden. Es ist aber auch möglich, von vornherein eine optisch aktive Ausgangssubstanz einzusetzen, wobei dann als Endprodukte eine entsprechende optisch aktive Form erhalten wird. Bei weiteren asymmetrischen C-Atomen erhält man diastereomere Gemische. Trennung kann nach den hierfür bekannten Methoden erfolgen.

Die vorliegende Erfindung umfasst also die Racemate und diastereomeren Formen sowie die entsprechenden optisch aktiven rechts-und linksdrehenden Formen.

Die Alkanoylreste in den Verbindungen der Formel I können solvolytisch wieder abgespalten werden, wodurch die entsprechenden Verbindungen der Formel I erhalten werden, die freie Hydroxygruppen beziehungsweise Aminogruppen haben. Diese solvolytische Abspaltung erfolgt beispielsweise durch Verseifung mit verdünnten Säuren oder mittels basischer Substanzen (Pottasche, Soda, wässrige Alkalilösungen, $NH_3$) bei Temperaturen zwischen 10 und 150° C, insbesondere 20 - 100° C.

Die erfindungsgemäßen Verbindungen sind zur Herstellung pharmazeutischer Zusammensetzungen beziehungsweise Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel kann unter Verwendung der bekannten und üblichen pharmazeutischen Trägermittel und Hilfsstoffe erfolgen.

Weitere pharmakologische und pharmazeutische Angaben

Die erfindugnsgemäßen Verbindungen zeigen im Asthmaversuch an wachen Meerschweinchen eine gute antiasthmatische und antiallergische Wirkung. Beipielsweise wird bei oben genannter Versuchsmehtode bei einer Dosis von 3 mg/kg Körpergewicht beim Meerschweinchen eine asthmaprotektive Wirkung gegen das allergische Asthma erzielt.

Die niedrigstge bereits wirksame Dosis in dem oben genannten Tierversuch ist beispielsweise

0,3 mg/kg oral
0,1 mg/kg intravenös

Als allgemeiner Dosisbereich für die Wirkung (Tierversuch wie oben) kommt beispielsweise in Frage:

0,1 - 30 mg/kg oral, insbesondere 1,0 - 10,-0 mg/kg
0,1 - 10 mg/kg intravenös, insbes. 0,3 - 5,0 mg/kg

Die Wirkungsrichtung der erfindungsgemäßen Verbindungen ist mit der Wirkung des bekannten Arzneimittelwirkstoffs Dinatriumcromoglicinsäure vergleichbar, jedoch bestehen hierzu insbesondere folgende Unterschiede: perorale Wirksamkeit, stärkere Wirksamkeit, lange Wirkungsdauer.

Indikationen für die die erfindungsgemäßen Verbindungen in Betracht kommen können: Allergische Rhinitis, Asthma bronchiale.

Die pharmazeutischen Zubereitungen enthalten im allgemeinen zwischen 0,1 bis 30 vorzugsweise 0,3 bis 10 mg der erfindunsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 1 und 10 mg oder Lösungen, die zwischen 1 bis 10 Gewichtsprozent an ativer Substanz enthalten.

Die Einzeldosis der erfindungsgemäßen aktiven Komponenten kann beispielsweise liegen

a) bei oralen Arzneiformen zwischen 0,1 - 30 mg, vorzugsweise 1 - 10 mg

b) bei parenteralen Arzneiformen (zum Beispiel intravenös, intramuskulär) zwischen 0,1 - 10 mg vorzugsweise 0,3 - 5,0 mg.

c) bei Arzneiformen zur Inhalation (Lösungen oder Aerosole) zwischen 1 - 10 mg vorzugsweise 3 - 5 mg - (Die Dosen sind jeweils bezogen auf die freie Base)

d) bei Arzneiformen zur rektalen oder vaginalen Applikation zwischen 0,5 - 50 mg vorzugsweise 1 - 30 mg

e) bei Arzneiformen zur lokalen Applikation auf die Haut und Schleimhäute (zum Beispiel in Form von Lösungen, Lotionen, Emulsionen, Salben und so weiter) zwischen 1 - 10 mg vorzugsweie 3 - 5 mg

Beispielsweise können 3mal täglich 1 bis 2 Tabletten mit einem Gehalt von 1,0 bis 10 mg wirksamer Substanz oder zum Beispiel bei intravenöser Injektion 1 bis 2mal täglich eine Ampulle von 1 bis 10 ml Inhalt mit 0,3 bis 5,0 mg Substanz empfohlen werden. Bei oraler Verabreichung ist die minimale tägliche Dosis beispielsweise 1 mg; die maximale tägliche Dosis bei oraler Verabreichung soll nicht über 30 mg liegen.

Für die Behandlung von Hunden und Katzen liegt die orale Einzeldosis im allgemeinen zwischen ungefähr 0,5 und 30 mg/kg Körpergewicht; die parenterale Dosis ungefähr zwischen 0,1 und 10 mg/kg Körpergewicht.

Für die Behandlung von Pferden und Vieh liegt die orale Einzeldosis im allgemeinen zwischen ungefähr 0,5 und 30 mg/kg; die parenterale Einzeldosis ungefähr zwischen 0,1 und 10 mg/kg Körpergewicht. -(Die Dosen sind jeweils bezogen auf die freie Base)-

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 250 und 1000 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

Beispiel 1

4-(p-Fluorbenzyl)-2-[2-(N-benzyl-N-methyl-amino)-ethyl]-1-(2H)-phthalazinon

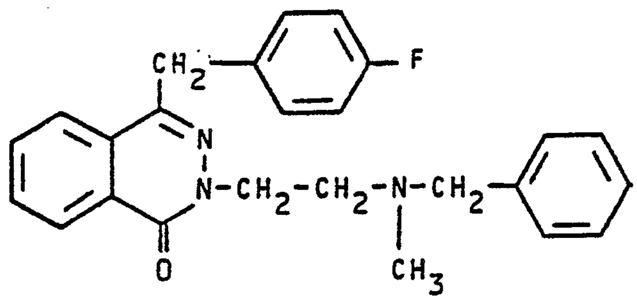

116,94 g (0,4 mol) des Kaliumsalzes des 4-(p-Fluorbenzyl)-1-(2H)-phthalazinons werden in 350 ml Dimethylacetamid suspendiert und durch Rühren gelöst. Zu dieser Lösung werden 55,10 g (0,3 mol) N-Benzyl-N-methyl-2-chlorethylamin gegeben (die freie Base wird aus dem entsprechenden Hydrochlorid durch Freisetzen mit $NH_3$ und Ausschütteln des Amins mit $CH_2Cl_2$, sowie anschließendem Eindampfen, gewonnen). Die Lösung wird für 5 Stunden auf 100°C erhitzt, wobei sich die anfänglich gelbe Lösung über orange bis hin zu tiefrot färbt. Das Dimethylacetamid wird abgezogen und der Rückstand in $CH_2Cl_2/H_2O$ aufgenommen Die organische Phase wird über $MgSO_4$ getrocknet, abfiltriert und dann eingedampft. Der Rückstand wird in 500 ml Aceton aufgenommen und mit 28,35 g (0,32 mol) wasserfreier Oxalsäure in 150 ml Aceton versetzt. Die über Nacht gebildeten Kristalle werden abgesaugt und getrocknet. Das Salz wird in 300 ml $H_2O$ aufgeschlämmt und mit 12,30 g (0,31 mol) NaOH in 150 ml $H_2O$ versetzt, dabei entsteht eine milchige Lösung. Im Scheidetrichter wird die Lösung mit 200 ml Ether geschüttelt und die organische Phase abgetrennt.

Die etherische Lösung wird über $MgSO_4$ getrocknet, abfiltriert und eingeengt. Der Rückstand wird in 150 ml Aceton gelöst und mit 40 ml 7,2 n isopropanolischer HCl (0,28 mol) versetzt. Bis zur leichten Trübung wird nun Ether zugegeben. Die über Nacht ausgefallenen weißen Kristalle werden abgesaugt und im Vakuum bei 50°C getrocknet.

Ausbeute: 105,22 g (60 % der Theorie)

Schmelzpunkt des Hydrochlorids: 183 - 184°C

Herstellung der Ausgangsstoffe

Kaliumsalz des 4-(p-Fluorbenzyl)-1-(2H)-phthalazinons

37,83 g (0,573 mol) KOH werden 450 ml Methanol gelöst und mit 143,17 g (0,56 mol) 4-(p-Fluorbenzyl)-1-(2H)-phthalazinon in der Wärme unter Rühren versetzt. Die klare Lösung wird am Vakuumrotationsverdampfer eingedampft, wobei weiße Kristalle zurückbleiben (F. über 250°C).

N-Benzyl-N-methyl-(2-chlor)-ethylamin

219,35 g (1,09 mol) N-Benzyl-N-methyl-(2-hydroxy)-ethylamin x HCl werden in 400 ml Chloroform gelöst und 54,6 ml (0,75 mol) Thionylchlorid zur Lösung zugetropft (setzt die Reaktion nicht ein, so werden einige Tropfen Dimethylformamid zum Starten zugegeben - die Reaktion ist durch die starke Gasentwicklung erkennbar). Nachdem die heftige Reaktion abgeklungen ist, wird die Lösung 2 Stunden am Rückfluß gekocht. Nach Zugabe von weiteren 40 ml (0,55 mol) $SOCl_2$ wird die Lösung für weitere 2 Stunde erhitzt.

Über Nacht falen weiße Kristalle aus, die abgesaugt, mit Ether verrührt und anschließend abgesaugt werden. Die Substanz wird in 400 ml Isopropanol umkristallisiert. Die weißen Kristalle des Hydrochlorids werden im Vakuum bei 50°C getrocknet. Ausbeute: 236,27 g (98 % der Theorie), Schmelzpunkt 139 - 140°C.

Das N-Benzyl-N-methyl-(2-hydroxy)-ethylamin x HCl wird zum Beispiel wie folgt erhalten:

Zu einer auf 0°C gekühlten Lösung von 167,76 g (1,38 mol) N-benzyl-methylamin in 280 ml Methanol gibt man auf einmal 82 ml (1,65 mol) Ethylenoxid. Die Lösung wird 1,5 Stunden bei 5°C gerührt. Dann wird das Eisbad entfernt (es ist keine exotherme Reaktion mehr zu beobachten) und für weitere 2 Stunden bei Raumtemperatur gerührt. Die Lösung wird am Vakuumrotationsverdampfer eingeengt, wobei ein gelblich, öliger Rückstand verbleibt. Dieser wird im Vakuum destilliert, wobei eine klare Flüssigkeit gewonnen wird: Siedepunkt 85 - 90°C unter 0,27 - 0,33 mbar. Ausbeute: 193,75 g (84 % der Theorie)

Die durch Destillation gereinigte Substanz wird in 1 Liter Diethylether gelöst und unter Kühlung mit 260 ml (1,3 mol) 7,2 n isopropanolischer HCl versetzt Das Lösungsmittel wird abgedampft und der Rückstand in Methanol aufgenommen. Die lösung wird abermals eingedampft, wobei eine gelblich, ölige Flüssigkeit zurückbleibt.

Die Beispiele 2 - 11 sind in der Tabelle 1 aufgeführt.

Die Beispiele besitzen die folgende Strukturformel A:

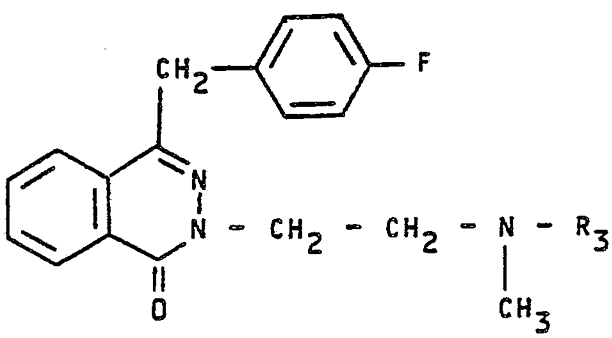

A

In Tabelle 1 ist in Spalte 2 ($R_3$) der Benzolring in der folgenden Form angegeben:

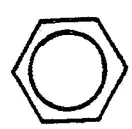

Tabelle 1

| Beispiel-Nummer | Rest $R_3$ | Salz | Schmelzpunkt |
|---|---|---|---|
| 2 | 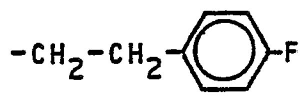 | x $H_2C_2O_4$ Oxalat | 155 - 156°C |
| 3 | 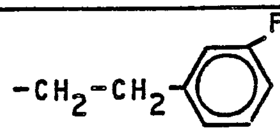 | x $H_2C_2O_4$ | 125 - 126°C |
| 4 | 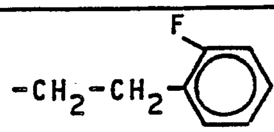 | x $H_2C_2O_4$ | 139 - 140°C |
| 5 | 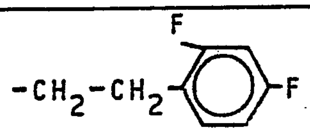 | x $H_2C_2O_4$ | 150 - 151,5°C |
| 6 | 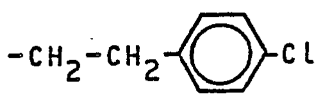 | x $H_2C_2O_4$ | 173 - 174°C |
| 7 | 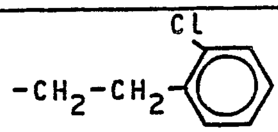 | x $H_2C_2O_4$ | 155 - 156°C |
| 8 | 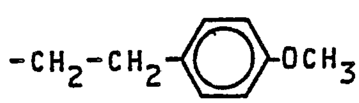 | x $H_2C_2O_4$ | 151 - 152°C |
| 9 | $-CH_2-C\equiv C-H$ | x HCl | 80 - 81°C |
| 10 | 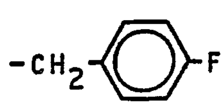 | x HCl | 136 - 137,5°C |
| 11 | 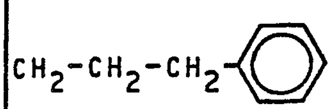 | x $H_2C_2O_4$ | 154 - 155,5°C |

| Beispiel-Nummer | Rest $R_3$ | Salz | Schmelzpunkt |
| --- | --- | --- | --- |
| 12 | $CH_2-CH_2-$ 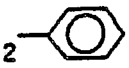 | $\times\ C_2H_2O_4$ | 143° C |
| 13 | $CH_2-CH=CH_2$ | $\times\ C_2H_2O_4$ | 139° C sintert ab 123° C |
| 14 | $CH_2-CH_2-OCH_3$ | $\times\ C_2H_2O_4$ | 116 - 118° C |

<u>Allgemeine Arbeitsvorschrift für die Beispiele 2 - 11</u>

Zur Umsetzung wird aus dem Hydrochlorid des 4-(p-Fluorbenzyl)-2-[2-(N-methylamino)-ethyl]-1-(2H)-phthalazinons (Formel A, wobei $R_3$ Wasserstoff ist) die Base freigesetzt. Dazu wird das Hydrochlorid in Wasser gelöst und mit der entsprechenden Menge konzentriertes $NH_3$ versetzt, wobei eine milchige Lösung entsteht. Die wässrige Lösung wird mit $CH_2Cl_2$ geschüttelt und die organische Phase über $MgSO_4$ getrocknet, abfiltriert und eingeengt, wobei die freie Base zurückbleibt. Das 4-(p-Fluorbenzyl)-2-[2-(N-methylamino)-ethyl]-1-(2H)-phthalazinon ( = Ausgangsphthalazinon) wird in p-Dioxan beziehungsweise Dimethylacetamid gelöst und mit der 3 - 4fachen Menge an Triethylamin beziehungsweise $K_2CO_3$ als Protonenfänger versetzt. Zu dieser Lösung gibt man die 2 - 5fache Menge des Alkylierungsmittels, das heißt der Substanz $R_3$-Z. Die reaktionsträgeren Chlorverbindungen bedürfen der extremeren Bedingungen mit $K_2CO_3$ in Dimethylacetamid, während die reaktiveren Bromide und p-Toluolsulfonsäure ester sich in p-Dioxan und mit Triethylamin als Protonenfänger gut umsetzen lassen. Die Lösung wird für die angegebene Reaktionszeit bis zum Siedepunkt 101 °C (p-Dioxan) beziehungsweise 110°C (Dimethylacetamid) erhitzt. Gebildetes Triethylammoniumhydrochlorid beziehungsweise Kaliumchlorid werden abfiltriert und die Lösung am Vakuumrotationsverdampfer eingeengt. Der verbleibende tiefrote bis braune Rückstand wird zwischen $CH_2Cl_2/H_2O$ verteilt. Die $CH_2Cl_2$-Phase wird über $MgSO_4$ getrocknet, abfiltriert und eingeengt. Das hierbei gewonnene Öl wird mittels Säulenchromatographie und/oder Salzbildung mit anschließender Umkristallisation gereinigt.

Salzbildung:

a) Hydrochlorid

Das gereinigte Öl wird in wenig Isopropanol aufgenommen und mit der entsprechenden Menge 7,2 n isopropanolischer HCl angesäuert (die Losung muß sauer reagieren). Anschließend wird Diethylether bis zur leichten, bleibenden Trübung zugesetzt und die Kristallisation über Nacht im Kühlschrank fortgesetzt. Durch Animpfen der Lösung mit zuvor im Reagenzglas gebildeten. Impfkristallen läßt sich der Kristallisationsvorgang unterstützen.

b) Maleate beziehungsweise Oxalate:

Das Öl wird in wenig Aceton aufgenommen und mit der entsprechenden Menge in Aceton gelöster wasserfreier Malein-beziehungsweise Oxalsäure versetzt. Bis zur leichten Trübung wird dann Diethylether zugegeben. Die Kristallisation wird über Nacht im Kühlschrank und gegebenenfalls durch Versetzen mit Impfkristallen durchgeführt.

Einzelheiten gehen aus der folgenden Aufstellung hervor:

Beispiel 2

Ansatz:
Ausgangsphthalazinon: 8,12 g (0,026 mol)
p-Fluorphenethylchlorid: 16,53 g (0,104 mol)
$K_2CO_3$: 3,75 g (0,027 mol)
Dimethylacetamid: 70 ml

Reaktionszeit: 18 Stunden
Reaktionstemperatur: 110°C
Aufarbeitung: Säulenchromatographie
Salzbildung: Oxalat
Ausbeute: 4,31 g (31 % der Theorie)

Beispiel 3

Ausgangsphthalazinon: 5,29 g (0,017 mol)
p-Toluolsulfonsäure-m-fluorphenethylester: 7,10 g (0,024 mol)
Triethylamin: 4,05 g (0,040 mol)
p-Dioxan: 80 ml

Reaktionszeit: 12 Stunden
Reaktionstemperatur: 101°C
Aufarbeitung: Umkristallisation in Methanol/Ether (1:1)
Salzbildung: Oxalat
Ausbeute: 1,92 g (21,5 % der Theorie)

Beispiel 4

Ansatz:
Ausgangsphthalazinon: 8,50 g (0,027 mol)
o-Fluorphenethylchlorid: 11,87 g (0,075 mol)
$K_2CO_3$: 3,51 g (0,029 mol)
Dimethylacetamid: 50 ml

Reaktionszeit: 21 Stunden
Reaktionstemperatur: 110°C
Aufarbeitung: Säulenchromatographie
Salzbildung: Oxalat
Ausbeute: 1,98 g (14 % der Theorie)

Beispiel 5

Ansatz:
Ausgangsphthalazinon: 8,53 g (0,027 mol)
o,p-Difluorphenethylchlorid: 9,88 g (0,056 mol)
$K_2CO_3$: 3,94 g (0,029 mol)
Dimethylacetamid: 100

Reaktionszeit: 25,5 Stunden
Reaktionstemperatur: 110°C
Aufarbeitung: Umkristallisation in Aceton/Ether (1:1)
Salzbildung: Oxalat
Ausbeute: 2,9 g (20 % der Theorie)

Beispiel 6

Ansatz:
Ausgangsphthalazinon: 9,34 g (0,030 mol)
p-Toluolsulfonsäure-p-chlorphenethylester: 17,43 g (0,058 mol)
Triethylamin: 7,08 g (0,070 mol)
p-Dioxan: 100 ml

Reaktionszeit: 11 Stunden
Reaktionstemperatur: 110°C
Salzbildung: Oxalat
Ausbeute: 7,83 g (48,3 % der Theorie)

Beispiel 7

Ansatz:
Ausgangsphthalazinon: 7,47 g (0,025 mol)
p-Toluolsulfonsäure-o-chlorphenethylester: 9,04 g (0,029 mol)
Triethylamin: 5,06 g (0,50 mol)
p-Dioxan: 100 ml

Reaktonszeit: 9 Stunden
Reaktonstemperatur: 101 °C
Aufarbeitung: Umkristalisaton in Methanol/Ether (1:1)
Salzbildung: Oxalat
Ausbeute: 2,08 g (16,3 % der Theorie)

Beispiel 8

Ansatz:
Ausgangsphthalazinon: 5,19 g (0,017 mol)
p-Toluolsulfonsäure-p-methoxyphenethylester: 6,13 g (0,02 mol)
Triethylamin: 4,05 g (0,04 mol)
p-Dioxan: 100 ml

Reaktionszeit: 6,5 Stunden

Reaktionstemperatur: 101 °C
Salzbildung: Oxalat
Aufarbeitung: Umkristallisation in Methanol/Ether (1:1)
Ausbeute: 2,87 g (31,8 % der Theorie)

Beispiel 9

Ansatz:
Ausgangsphthalazinon: 6,85 g (0,022 mol)
3-Brompropin: 3,09 g (0,026 mol)
Triethylamin: 5,06 g (0,050 mol)
p-Dioxan: 100 ml

Reaktionszeit: 7 Stunden
Reaktionstemperatur: 101 °C

Die freie Base fällt nach der Aufarbeitung kristallin an und wird durch Verreiben mit Ether und anschließendem Abfiltrieren gereinigt. Ein Dünnschichtchromatogramm ergibt noch 5 - 6 % Verunreingungen. Zur Reinigung wird aus der freien Base das hydrochlorid gebildet und umkristallisiert.

Ausbeute: 1,80 g (21,2 % der Theorie)

Beispiel 10

Ansatz:
Ausgangsphthalazinon: 6,23 g (0,02 mol)
p-Fluorbenzylchlorid: 5,78 g (0,04 mol)
Triethylamin: 6,07 g (0,06 mol)
p-Dioxan: 150 ml

Reaktionszeit: 10 Stunden
Reaktionstemperatur: 101
Salzbildung: als Hydrochlorid
Aufarbeitung: Umkristallisieren in Isopropanol/Ether (1:1)
Ausbeute: 3,71 g (40,5 % der Theorie)

Beispiel 11

Ansatz:
Ausgangsphthalazinon: 6,23 g (0,02 mol)
$\gamma$-Phenylpropylbromid: 8,96 g (0,045 mol)
Triethylamin: 6,07 g (0,06 mol)
p-Dioxan: 150 ml

Reaktionszeit: 16 Stunden
Reaktionstemperatur: 101 °C
Salzbildung: Oxalat
Aufarbeitung: Umkristallisation in Methanol/Ether (1:1)
Ausbeute: 2,25 g (21,5 % der Theorie)

Beispiel 12

Ansatz:
Ausgangsphthalazinon: 7.5 9 (24.1 mmol)
Phenethylbromid: 10,7 g (58,1 mmol)
Triethylamin: 10 ml (71,8 mmol)
Dioxan: 70 ml

Reaktonszeit: 16 Stunden
Reaktonstemperatur: 130°C
Aufarbeitung: Auskristallisation aus Aceton durch Etherzusatz
Ausbeute: 6,0 g (49,3 % der Theorie)

Beispiel 13

Ansatz:
Ausgangsphthalazinon: 6,95 g (22,3 mmol)
Allylbromid: 3,50 g (28,9 mmol)
Triethylamin: 7 ml (50,3 mmol)
Dioxan: 60 ml

Reaktionszeit: 10 Stunden
Reaktionstemperatur: 85° C
Aufarbeitung: Auskristallisation aus Aceton durch Etherzusatz
Ausbeute: 3,36 g (34,1 % der Theorie)

Beispiel 14

8,15 g (26,2 mmol) Ausgangsphthalazinon werden in 50 ml Dimethylacetamid gelöst und mit 3,79 g (27,4 mmol) $K_2CO_3$ versetzt. Man pipettiert 7,5 ml (82,3 mmol) 1-Chlor-2-methoxyethan zu der Lösung und kocht diese 18 Stunden bei 110° C unter Rückfluß. Das abgekühlte Gemisch wird eingeengt (Rotationsverdampfer) zwischen $CH_2Cl_2/H_2O$ verteilt und die Phasen getrennt. Die organische Phase wird über $MgSO_4$ getrocknet, abiltriert une das Filtrat eingeengt. Das resultierende Öl wird über eine Kieselgelsäule chromatographiert (Elutionsmittel: $CH_2Cl_2$/Methanol/25% $NH_3$ = 90/9/1). Die entsprechenden Fraktionen werden eingedampft und das Salz, wie für die anderen Beispiele angegeben ist, gebildt.

Ausbeute: 1,2 g (10,0 % der Theorie)

Das Ausgangsphthalazinon wird zum Beispiel wie folgt hergestellt:

4-(p-Fluorbenzyl)-2-[2-(N-methyl-aminoethyl)]-1-(2H)-phthalazinon-Hydrochlorid

105,22 g (0,24 mol) 4-(p-Fluorbenzyl)-2-[2-(N-benzyl-N-methyl-amino)-ethyl]-1-(2H)-phthalazinon x HCl (Beispiel 1) werden in 500 ml Methanol gelöst und mit 7,26 g 10 % Pd auf Aktivkohle, in 40 ml Ethanol suspendiert, versetzt. Diese Lösung wird eine Stunde bei Atmosphärendruck hydriert, wobei 6,300 Liter H2 (theoretischer Verbrauch: 5,856 Liter $H_2$) verbraucht werden. Die Aktivkohle wird abfiltriert und die Lösung eingeengt. Der Rückstand wird bis zur leichten Trübung mit Ether versetzt. Über Nacht bilden sich Kristalle,

die abgesaugt werden. Die weißen Kristalle werden in 100 ml siedendem Ethanol gelöst und die abgekühlte Lösung mit Ether bis zur leichten Trübung versetzt. Die entstandenen weißen Kristalle des 4-(p-Fluorbenzyl)-2-[2-(N-methyl-amino)-ethyl]-1-(2H)-phtalazinon-Hydrochlorids werden abgesaugt und bei 50°C im Vakuum getrocknet. Ausbeute: 78,71 g (95,8 % der Theorie) Schmelzpunkt des Hydrochlorids: 139 - 141,5°C.

Beispiel 15

4-(p-Fluorbenzyl)-2-[2-dibenzylamino-ethyl]-1-2H-phthalazinon

25,4 g (0,10 mol) 4-(p-Fluorbenzyl)-1-2H-phthalazinon werden in 100 ml Ethanol gelöst und 42,6 ml (0,11 mol) 20%iges Na-Ethanolat zugetropft. Das Ethanol wird abgedampft, der Rückstand mit Dioxan versetzt und auf 70°C erwärmt. Bei dieser Temperatur gibt man N-(2-Chlorethyl)-dibenzylamin, gelöst in Dioxan, hinzu, wobei sich Kochsalz abscheidet. Das Reaktionsgemisch wird nach weiterem 3stündigem Erhitzen bei dieser Temperatur vom NaCl abgesaugt, das Filtrat am Rotationsverdampfer eingeengt und mit Methylenchlorid aufgenommen. Diese Lösung wird 3mal mit 10%iger HCl und 1mal mit Bicarbonatlösung ausgeschüttelt, um nach dem Eindampfen die Base mit $CH_2Cl_2$/NaOH wieder freizusetzen. Das Produkt kristallisiert nach Zugabe von Diethylether. Ausbeute: 31,0 g (65 %). F.: 92-94°C.

Beispiel 16

4-(p-Fluorbenzyl)-2-[2-(N-ethyl-N-phenethyl-amino)-ethyl]-1-(2H)-phthalazinon

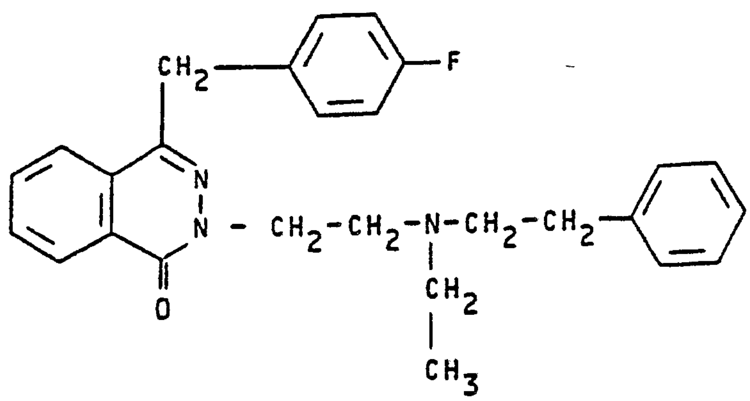

Allgemeine Arbeitsvorschrift

Aus dem Hydrochlorid des N-Ethyl-N-phenethyl-2-chlorethylamins wird die Base freigesetzt und in Dimethylacetamid gelöst. Zur Lösung wird im Überschuß das Kaliumsalz des 4-(p-Fluorbenzyl)-1-(2H)-phthalazinons zugegeben. Die Lösung wird für die angegebene Reaktionszeit auf 110°C erhitzt und gerührt. Nach der Reaktionszeit wird die Lösung am Vakuumrotationsverdampfer eingeengt und der verbleibende Rückstand zwischen $CH_2Cl_2$/$H_2O$ verteilt. Die organische Phase wird über $MgSO_4$ getrocknet, abfiltriert und eingeengt. Der verbleibende Rückstand wird in Aceton gelöst und das ausgefallene 4-(p-Fluorbenzyl)-1-(2H)-phthalazinon abfiltriert oder die Lösung in einem basischem Laufmittel (90 Volumen% $CH_2Cl_2$, 10 Volumen% Methanol, 1 Volumen% konzentriertes Ammoniak) an einer Kieselgelsäule gereinigt. Die so vorgereinigte Substanz wird zum Hydrochlorid oder Oxalat umgesetzt, umkristallisiert und getrocknet.

Ansatz:
K-Salz des 4-(p-Fluorbenzyl)-1-(2H)-phthalazinons: 7,36 g (0,025 mol)
N-Ethyl-N-phenethyl-2-chlor-ethylamin: 3,39 g (0,016 mol)
Dimethylacetamid: 70 ml
Reaktionszeit: 8 Stunden
Reaktionstemperatur: 110°C
Aufarbeitung: Das 4-(p-Fluorbenzyl)-1-(2H)-phthalazinon wird aus Aceton ausgefällt und abfiltriert.
Salzbildung: In Isopropanol wird der Rückstand aufgenommen und mit 2,5 ml 7,2 n isopropanolischer HCl versetzt und das gleiche Volumen an Ether zugegeben.

Ausbeute: 2,89 g (38,76 % der Theorie)
F. des Hydrochlorids: 178 - 179,5°C

Beispiel 17

4-(p-Fluorbenzyl)-2-[2-(N-cyclopentyl-N-methyl-amino)-ethyl]-1-(2H)-phthalazinon

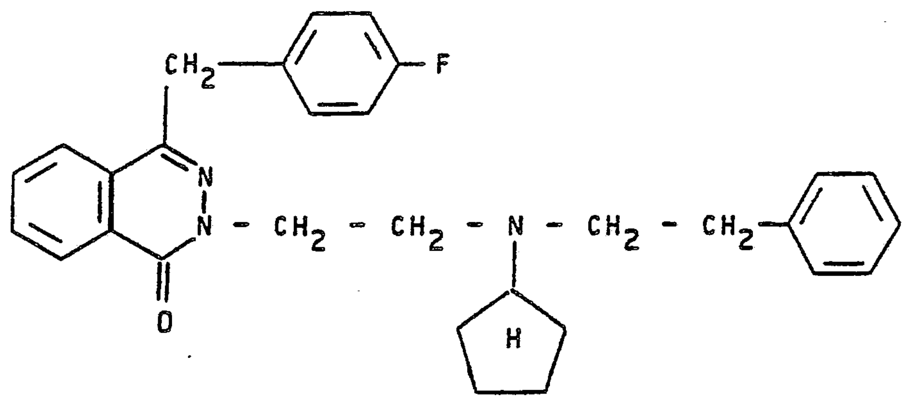

Die Herstellung erfolgt analog Beispiel 16.

Ansatz:
K-Salz des 4-(p-Fluorbenzyl)-1-(2H)-phthalazinons: 2,78 g (0,0095 mol)
N-β -Phenethyl-N-cyclopentyl-2-chlor-ethylamin: 2,14 g (0,0085 mol)
Dimethylacetamid: 60 ml
Reaktionszeit: 8 Stunden
Reaktionstemperatur: 110°C
Aufarbeitung: Säulenchromatographie
Salzbildung: Als Oxalat
Ausbeute: 1 g
Das Oxalat schmilzt bei 155 - 156°C

Beispiel 18

4-(p-Fluorbenzyl)-2-[2-(N-cyclohexyl-N-β-phenethylaminoethyl)]-1-(2H)-phthalazinon

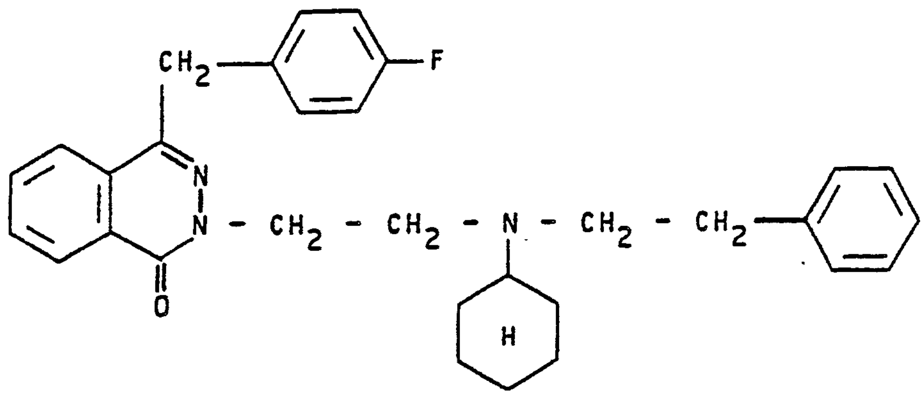

Die Herstellung erfolgt analog Beispiel 16.

Ansatz:
K-Salz des 4-(p-Fluorbenzyl)-1-(2H)-phthalazinons: 4,49 g (0,015 mol)
N-Cyclohexyl-N-β-phenethyl-2-chlor-ethylamin: 2,66 g (0,010 mol)
Dimethylacetamid: 60 ml
Reaktionszeit: 12 Stunden
Reaktionstemperatur: 110°C
Aufarbeitung: Säulenchromatographie
Salzbildung: Als Oxalat
Ausbeute: 1,10 g (19,2 % der Theorie)
Das Oxalat sintert bei 89 - 90°C.

Beispiel 19 (Abspaltung ner Benzylgruppe)

4-(p-Fluorbenzyl)-2-(2-benzylamino-ethyl)-1-2H-phthalazinon

Zu 15,4 g (0,032 mol) in 100 ml Ethanol gelöstem 4-(p-Fluorbenzyl)-2-[2-dibenzylamino-ethyl]-1-2H-phthalazinon gibt man 1,5 g 10%iges Pd auf Aktivkohle sowie 5 ml 10%ige HCl. Nach dem Spülen der Apparatur mit Wasserstoff wird 20 Minuten bei 40°C hydriert, wobei 810 ml $H_2$ (berechnetes Volumen: 774 ml) aufgenommen werden. Vom Katalysator wird noch heiß abgesaugt und das Filtrat am Rotationsverdampfer eingeengt. Der Abdampfrückstand wird nach dem Ansäuern mit isopropanolischer HCl aus Toluol/methylethylketon umkristallisiert. Das abgesaugte Produkt wird in 10%iger HCL aufgeschlämmt und in $CH_2Cl_2$ geschüttelt. Nach dem Trocknen über $Na_2SO_4$ wird der eingedampfte Rückstand aus Isopropanol umkristallisiert, abgesaugt und im Vakuum bei 50°C getrocknet.
Ausbeute: 11,2 g (81,4 %)
F. des Hydrochlorids: 151 - 154°C

Beispiele für pharmazeutische Zubereitungen

Kapseln enthaltend 5 mg Verbindung gemäß Beispiel 1

5 g Wirkstoff nach Beispiel 1 werden mit 111, 7 g Calciumhydrogenphosphat durch einen Sieb gegeben (0,8 mm Maschenweite) und mit einer Lösung aus 2,3 g Gelatine und 0,1 g Polysorbat 80 in 22,6 g Wasser angefeuchtet, durch einen Sieb der Maschenweite 2 mm granuliert und bei 40° C getrocknet. Das trockene Granulat und 20,9 g Maisstärke passiert man durch einen 0,8-mm-Sieb und homogenisiert. Diese Masse wird auf einer geeigneten Kapselmaschine zu 140 mg in Hartgelatinekapseln der Größe 3 abgefüllt.
1 Kapsel enthält 5 mg Wirkstoff nach Beispiel 1.

Injektonslösung (intravenös), enthaltend 2,5 mg/ml Verbindung gemäß Beispiel 8

2,5 g Wirkstoff nach Beispiel 8 und 9 g Kochsalz werden unter Rühren in 800 g Wasser für Injektonszwekce gelöst und auf 1 Liter aufgefüllt. Unter aseptischen Bedingungen wird diese Injektionslösung durch Filtration über keimdichte Filter (0,2 µm Porenweite) sterilisiert. Schließlich füllt man unter aseptischen Bedingungen und unter Stickstoffbegasung 1,1 ml (Nennvolumen 1 ml) in sterile 2 ml Ampullen ab.
1 Ampulle enthält 2,5 mg Wirkstoff nach Beispiel 8.

**Ansprüche**

1. 2-Aminoalkyl-4-benzyl-1-(2H)-phthalazinon-Derivate der Formel

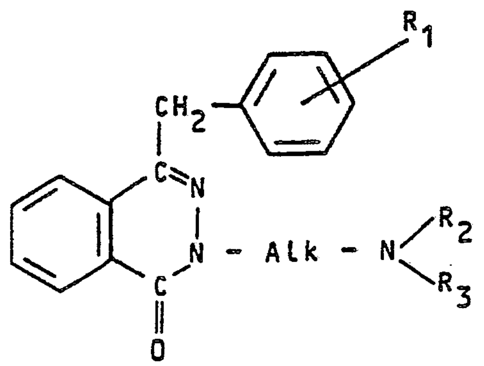

I

worin $R_1$ Wasserstoff, Fluor, Chlor, Brom, Trifluormethyl, $C_1$-$C_6$-Alkyl oder $C_1$-$C_6$-Alkoxy bedeuted, $R_2$ Wasserstoff, Phenyl, Benzyl, $C_1$-$C_6$-Alkyl oder $C_3$-$C_8$-Cycloalkyl bedeuted, $R_3$ einen $C_1$-$C_6$-Alkoxy-$C_1$-$C_6$-alkylrest, einen $C_3$-$C_8$-Cycloalkylrest, einen $C_3$-$C_6$-Alkenylrest, einen $C_3$-$C_6$-Alkinylrest, einen Phenylrest oder einen Phenyl-$C_1$-$C_6$-alkylrest darstellt, der im Phenylteil gegebenenfalls durch Halogen, $NO_2$, $NH_2$, CN, OH, $C_1$-$C_6$-Alkyl, $C_1$-$C_6$-Alkoxy, $C_2$-$C_6$-Alkanoyloxy oder $C_2$-$C_6$-Alkanoylamino substituiert ist und
Alk eine $C_2$-$C_6$-Alkylenbrücke darstellt, die auch eine Doppelbindung enthalten kann
und deren physiologisch unbedenkliche Säureadditionssalze.

2. Verfahren zur Herstellung von 2-Aminoalkyl-4-benzyl-1-(2H)-phthalazinon-Derivaten der Formel

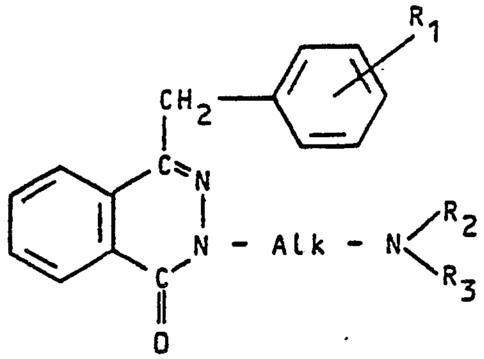

I

worin $R_1$ Wasserstoff, Fluor, Chlor, Brom, Trifluormethyl, $C_1$-$C_6$-Alkyl oder $C_1$-$C_6$-Alkoxy bedeuted, $R_2$ Wasserstoff, Phenyl, Benzyl, $C_1$-$C_6$-Alkyl oder $C_3$-$C_8$-Cycloalkyl bedeuted, $R_3$ einen $C_1$-$C_6$-Alkoxy-$C_1$-$C_6$-alkylrest, einen $C_3$-$C_8$-Cycloalkylrest, einen $C_3$-$C_6$-Alkenylrest, einen $C_3$-$C_6$-Alkinylrest, einen Phenylrest oder einen Phenyl-$C_1$-$C_6$-alkylrest darstellt, der im Phenylteil gegebenenfalls durch Halogen, $NO_2$, $NH_2$, CN, OH, $C_1$-$C_6$-Alkyl, $C_1$-$C_6$-Alkoxy, $C_2$-$C_6$-Alkanoyloxy oder $C_2$-$C_6$-Alkanoylamino substituiert ist und Alk eine $C_2$-$C_6$-Alkylenbrücke darstellt, die auch eine Doppelbindung enthalten kann und deren physiologisch unbedenklichen Säureadditionssalzen, dadurch gekennzeichnet, daß man

a) eine Verbindung der allgemeinen Formel

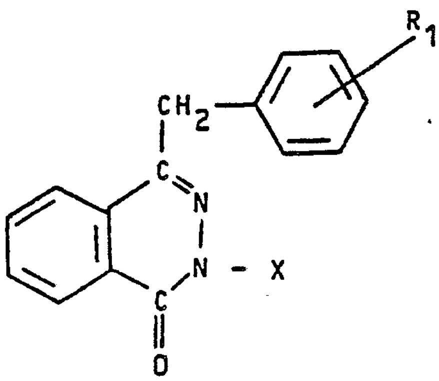

II

worin $R_1$ a die angegebenen Bedeutungen hat und X Wasserstoff, die Gruppe -Alk-$NHR_2$, die Gruppe -Alk-$NHR_3$ oder die Gruppe -Alk-Y ist, wobei $R_2$ und $R_3$ die angegebenen Bedeutungen haben und Y ein Halogenatom oder eine durch eine starke anorganische oder organische Säure veresterte Hydroxygruppe darstellt und die Verbindung II auch in Form eines Metallsalzes eingesetzt werden kann, falls X Wasserstoff ist, mit einer Verbindung der Formel

Z - W III

umsetzt, worin Z jeweils von X und Y verschieden ist und Wasserstoff oder eine durch eine starke anorganische oder organische Säure veresterte Hydroxygruppe darstellt und W der Rest $R_2$ oder der Rest $R_3$, die Gruppe -Alk-$NR_2R_3$ oder die Gruppe -$NR_2R_3$ ist und Alk jeweils die angegebene Bedeutung hat, in den erhaltenen Verbindungen gegebenenfalls vorhandene Benzylgruppen und/oder vorhandene Alkanoylreste abspaltet und gegebenenfalls die erhaltenen Verbindungen in ihre Säureadditionssalze überführt, oder

b) eine Verbindung der Formel

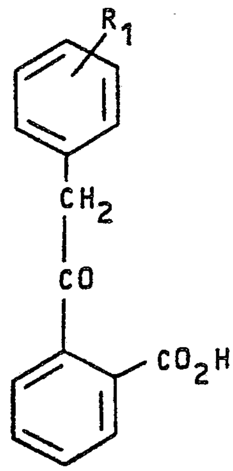

IV

oder ein reaktionsfähiges Derivat derselben, worin $R_1$ die angegebenen Bedeutungen hat, mit einem Hydrazin-Derivat der allgemeinen Formel

$H_2N - NH - Alk - NR_2R_3$ V

worin Alk, $R_2$ und $R_3$ die angegebenen Bedeutungen haben, umsetzt, gegebenenfalls vorhandene Alkanoylreste und/oder vorhandene Benzylgruppen abspaltet, und gegebenenfalls die erhaltenen Verbindungen in ihre Säureadditionssalze überführt.

3. Verbindungen der Formel I zur Anwendung als therapeutische Wirkstoffe.

4. Arzneimittel, enthaltend eine Verbindung der allgemeinen Formel I neben üblichen Träger-und/oder Verdünnungs-beziehungsweise Hilfsstoffen.

5. Verfahren zur Herstellung eines Arzneimittels,
dadurch gekennzeichnet,
daß eine Verbindung der allgemeinen Formel I mit gebräuchlichen pharmäzeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I zur Herstellung von Arzneimitteln.